# EUROPEAN PATENT SPECIFICATION

(11) **EP 3 836 781 B1**
(45) Date of publication and mention of the grant of the patent: **06.11.2024**
(21) Application number: 19849828.9
(22) Date of filing: 15.08.2019
(51) Int. Cl.: A01K 29/00, A01K 11/00, A61B 5/20, A61B 5/00, A61B 7/00, G01F 9/00, G06N 3/04, G06N 5/00, G10L 25/21, G10L 25/51, G10L 25/18

(54) **A METHOD, APPARATUS AND SYSTEM FOR DETECTING URINATION EVENTS FOR LIVESTOCK**
VERFAHREN, VORRICHTUNG UND SYSTEM ZUR ERKENNUNG VON URINIERUNGSEREIGNISSEN FÜR VIEH
PROCÉDÉ, APPAREIL ET SYSTÈME DE DÉTECTION D'ÉVÉNEMENTS DE MICTION POUR ANIMAUX D'ÉLEVAGE

(30) Priority: 17.08.2018 NZ 18745410
(43) Date of publication of application: 23.06.2021
(73) Proprietor: Agresearch Limited, Hamilton 3204 (NZ)
(72) Inventor: SHORTEN, Paul Robert, Hamilton (NZ); WELTEN, Brendon, Hamilton (NZ)
(74) Representative: Evens, Paul Jonathan
(86) International application number: PCT/NZ2019/050098
(87) International publication number: WO 2020/036496

(56) References cited:
- WO-A1-2015/088345
- WO-A1-2016/030875
- WO-A1-2017/087363
- WO-A2-2006/015372
- WO-A2-2006/015372
- WO-A2-2014/188273
- US-A1- 2014 155 756

## Description

### Field of Invention

The present invention relates to an apparatus, system and methods of detecting urination events for livestock animals. The invention has particular application to non-invasive techniques of detecting urination events for livestock animals such as dairy cows by using acoustic sensors.

### Background to the Invention

The implications of excessive nitrogen being excreted in urine by grazing livestock such as dairy cows and sheep onto farmed land is well known. Dairy farm systems in particular are prone to high nitrogen losses, largely due to only about 10 to 30% of the nitrogen consumed by a dairy cow being converted to products (in the form of milk, meat, fibre). The remainder of the consumed nitrogen is primarily excreted in urine and deposited onto the soil in concentrated areas at high nitrogen application rates equivalent to about 500 to 1,200 kilograms nitrogen per hectare.

The nitrogen derived from this urine is well in excess of that required by pasture plants and after transformations in soil, it is prone to being lost via leaching or gaseous emissions pathways. Nitrogen derived from the urine of grazing animals has been found to contribute significantly to nitrous oxide emissions, ammonia emissions and nitrate leaching to groundwater. Therefore, a good opportunity exists to reduce nitrogen losses from grazed pasture land by specifically targeting individual urine patches.

There is evidence that cows exhibit repeatable phenotypic variation in urination event volume and the frequency of urinations events per day. This difference in urination event volume and daily urination frequency between individual cows can be as much as two-fold. Furthermore, the nitrogen load of an individual urination event is also highly correlated with the time from the previous event, the time of day and the duration of the urination event.

This means that cows which urinate more frequently per day coupled with a lower volume per urination event tend to excrete lower amounts of nitrogen per urination event and thus represent a lower risk to the environment. This is because pasture plants in the urine "patch" (the area of pasture onto which urine is discharged) can utilise a greater proportion of the urine nitrogen for plant growth when urine is deposited at a lower nitrogen application rate.

Conversely, for cows that have less urination events per day coupled with a greater volume per urination event, the amount of nitrogen excreted in the urine patch is higher; a greater amount of the nitrogen is unable to be utilised by pasture plants and therefore this nitrogen is vulnerable to being lost to gaseous emissions and/or being leached into waterways.

As there is little variance between individual cows in terms of the urine flow rate, knowing the duration of the urination event allows the volume of the urination event to be calculated based on existing calibration curves from urine sensor studies that indicate that urine volume is proportional to the duration of the urination event.

Even though individual cows may excrete similar total amounts of nitrogen in urine over a period of 24-hours irrespective of whether they urinate, for example 12 times a day versus 18 times a day, it is preferable when considering minimising urine nitrogen loading on pasture and/or losses of nitrogen from grazed pasture land, that a dairy herd contain cows that urinate more frequently per day with a lower volume per urination event resulting in lower amounts of nitrogen per urination event.

However, there can be variance in the urination nitrogen load between individual cows and individual urination events for a particular cow. This means that a single urination event should not be relied upon for determining the impact on nitrogen loading by a specific cow. It would be preferable to make this determination based on a series of urination events for greater accuracy.

Being able to measure the frequency and duration of individual urination events over a specified period of time to determine urination frequency, average volume of urine excreted per urination event, and thus the amount of nitrogen per urination event, can assist in making appropriate management decisions, both at herd level and for individual cows.

The best way of achieving this is to measure the duration of individual urination events, preferably over a period of time covering at least 72 hours, to determine an average for the individual cow.

However, in practice, it is difficult to monitor the time, frequency and duration of individual urination events.

One method may involve manually observing the cow in the field and timing the length of any urination events that may occur. The longer the period of time the cow is observed, the more urination events are observed. Thus, a more accurate average for the duration of the urination event and therefore the volume of urine excreted in that event, can be determined. A more accurate estimate of urination frequency (events/day) can also be obtained.

Cows typically urinate 12 times in a 24-hour period. This means it is not good practice to monitor the animal for a limited period of time, for example across three hours per day where cows would be expected to urinate only one to two times. It would be preferable that any such monitoring take place over a 24 to 72-hour period. While this may mean more accurate determination of the timing of the urination event, and estimation of any diurnal time patterns in frequency and duration of urination events, it is clearly time-consuming and not practical, particularly when dealing with more than one animal.

This approach also requires a clear field of continuous observation; this may be difficult to achieve in practice as the cow of interest is typically in close proximity to other cows of the herd. Of course, the individual cow being monitored could be isolated from the herd but this may affect its behaviour and lead to changes in patterns, such as frequency of urination events that could affect the accuracy and therefore potentially bias any determinations of urination parameters for that cow.

An alternative to this would be to use sensors or other equipment placed into or proximate to the urinary tract. This would do away with the need for a person to watch one or more cows for extended periods of time and allow it to retain, as much as is possible, its normal behaviour.

However, care would need to be taken when securing sensors (some of which may require adhesive patches or the like to hold the sensing equipment and associated paraphernalia in place). It can also be difficult to apply invasive sensors to the urinary tracts of cows for many will exhibit avoidance behaviours. Placement of invasive sensors for detecting urination events, some having a weight of 500 to 1,500 grams, can take considerable time. Care would need to be taken that the cow, or the person attempting to locate the sensor, is not injured when attaching, inserting or checking the operation of the sensor. This provides an extra cost and constraint to phenotyping larger numbers of animals.

There is also the difficulty of ensuring that the sensors remain in situ for an appropriate period of time. In a typical farm environment, the sensors may become detached, dislodged or malfunction as the animal gets up and down, generally ambulates around the farm or knocks into farm/milking shed, fences, races or posts.

Cows can also generate large amounts of mucus that can block or hinder the operation of the urine sensors. A sustained high-mucus confined environment can lead to cow discomfort and/or infection.

Furthermore, it will be appreciated that cows are exposed to considerable amounts of fluids, in the form of rainfall, condensation, mud, dung, and urine, all of which have the potential to affect the properties of adhesives that may be used to secure some types of sensing equipment.

Another method may involve the use of indoor metabolism stalls or similar facilities to house cows. These are constructed with sensors and/or collection devices or the like that are arranged to detect and collect the excretions of the cow. Furthermore, this method is not practical when dealing with large dairy herds, which can comprise hundreds of individual cows. It would entail considerable expense for stalls to be built for all the individuals of the herd, so in practice, only a limited number of stalls would be available.

The farmer would need to select cows to go into these stalls for a period of time, diverting them as appropriate as they transit through the dairy farm. This would then require the farmer to avoid inadvertently selecting a previously tested cow to go into the stalls which can be difficult when dealing with a large dairy herd. This process would require careful management of the herd and may be potentially very time consuming.

For temperate grazed pasture systems whereby cows graze outdoors, this approach may not be representative of normal grazing behaviours that the cow would experience in its daily life. For example, the cow is typically offered harvested feed in portable bins and animal movements are restricted when in indoor metabolism stalls. Care must then be taken when extrapolating observations derived from such indoor metabolism stalls to outdoor field conditions. In addition, metabolism stalls do not typically provide information on individual urination events; instead they provide aggregate information typically on a daily basis (e.g. total volume of urine excreted per day).

As already noted, dairy cows can excrete similar total amounts of nitrogen in urine on a daily basis but can exhibit large variations between cows in the amount of nitrogen excreted on a per urination event basis. This variation can be harnessed to minimise the environmental impact of grazing livestock.

WO2015088345 discloses a urinating detector for an animal housing which includes a microphone and processing unit. RFID and/or video/image information is used to determine the identity of the animal urinating from a group of animals.

WO006015372 relates to a monitoring system for animal husbandry, and discloses animal-borne sensors, which optionally include acoustic sensors, and the presence of urination may be monitored.

WO2017087363 relates to animal wearable devices, systems and methods, and discloses an animal wearable device which may be used to determine if the animal is or is not about to urinate using *inter alia* sound received from a microphone component.

### Object of the Invention

It is an object of the invention to provide an apparatus, system and method for detecting a urination event for a livestock animal.

There is a desire to provide an apparatus, system and method for non-invasive measurement of the duration of urination event in one or more livestock animals.

There is also a desire to provide an apparatus, system and method for non-invasive measurement of the time and frequency of urination events in one or more livestock animals.

There is also a desire to provide an apparatus, system and method for determining the extent of nitrogen loading on land that is derived from the urine of one or more livestock animals.

There is also a desire to provide a user with a tool for managing a herd of livestock animals to minimise its impact on nitrogen loading on land.

There is also a desire to provide an apparatus, system and method for non-invasive measurement of the diurnal and seasonal variation in the frequency, duration and volume of urination events in one or more livestock animals.

### Summary of the Invention

In accordance with the present invention, there is provided: a method of detecting a urination event for a livestock animal as defined in appended independent claim 1; an apparatus suitable for using the method of detecting a urination event for a livestock animal, the apparatus being defined in appended claim 8; and a system suitable for using the method of detecting a urination event for a livestock animal, the system being defined in appended claim 15. Embodiments of the present invention are defined in appended claims dependent on any of claims 1, 8 and 15.

According to a first aspect of the invention, there is provided a method of detecting a urination event for a livestock animal, wherein the livestock animal has two rear legs, the method including the steps of:
1. placing a first acoustic sensor on a first rear leg;
2. monitoring for an audio signal of specific sound intensity and/or spectral signature;
3. measuring the duration of the audio signal; and
4. determining whether the duration of the audio signal corresponds to a urination event.

According to a further aspect of the invention, there is provided an apparatus suitable for using the method of detecting a urination event for a livestock animal according to the first aspect,and wherein the apparatus includes:
an acoustic sensor, wherein the sensor is configured to be placed on a rear leg of a livestock animal, and wherein the sensor includes:
a data collection device; and
a processor, wherein the processor is configured to record data relating to an audio signal of specific sound intensity, spectral signature, and duration.

According to a further aspect of the invention, there is provided a system suitable for using the method of measuring a urination event for a livestock animal according to the first aspect, and wherein the system includes:
the above apparatus aspect of the invention; and
a processor configured to correlate audio signals of specific sound intensity, spectral signature, and duration to criteria defining a urination event and determine whether a urination event has occurred for said livestock animal.

The invention is a method, apparatus and system for detecting the duration of urination events in livestock animals such as cows. An embodiment of the invention detects audio signals of specific intensity and/or spectral signature made by the cow as it urinates, monitors the duration of these sounds, and correlates these to criteria defining a urination event. From this, the approximate volume of urine excreted by that cow during the urination event can be determined. Over a period of time, an average for urine excreted per urination event can be calculated for the cow. The time and frequency of urination can also be determined from this invention. The time, duration and time elapsed from a previous urination event can be used to estimate the nitrogen load of a urination event.

This information can be used to assist in management decisions relating to the cow. For example, based on the nitrogen load of an animal, it may be removed from the dairy herd. This information can also be used to provide information on the genetic correlations and heritability of urination frequency, time of urination and urination duration. This information can also be used to associate cows with high urine nitrogen load to soil, pasture and land class that is more able to utilise the excreted nitrogen load.

A livestock animal should be understood to be quadruped animals kept and raised for their value as commodities, either through their flesh (as meat) or from their by-products (milk, hides, wool).

Reference will now be made throughout the remainder of the present specification to the livestock animal being a dairy cow, i.e. cows kept for their production of milk. However, this is not meant to be limiting and the present invention may be used with other ruminant species kept as livestock including, but not limited to, sheep, goats, beef cows and so on.

This method involves the use of an apparatus in the form of an acoustic sensor. This should be understood to be a sensor that measures and records audio signals that are indicative of properties of sound.

In exemplary embodiments of the invention the acoustic sensor measures and records more than one type of audio signal.

The acoustic sensor measures sound intensity. This should be understood to be the power carried by sound waves per unit area in a direction perpendicular to that area, measured in watt per square meter (W/m²).

The acoustic sensor also preferably measures the sound pressure level (measured in Pascals), sound power level (measured in Watts), sound pressure level (measured in decibels (dB or dB SPL (sound pressure level))), or sound power level (measured in decibels (dB or dB SWL (sound power level))) at a frequency (measured in Hertz (Hz) or kilohertz (kHz)). This list is not meant to be limiting and it should be appreciated that audio signals relating to other sound or noise properties may be measured. Measuring multiple sound properties allows for improved accuracy in determining whether a certain sound corresponds with those of a urination event.

In exemplary embodiments of the invention, the acoustic sensor is an audio recording device in the form of a microphone and shall be referred to as such throughout the remainder of the present specification. A microphone converts sound to electrical energy and also includes a processor to record the audio signal and convert it to a digital form.

However, the use of a microphone as the acoustic sensor is not meant to be limiting and persons skilled in the art will appreciate other types of acoustic sensors may be used. For example, the acoustic sensor may be a digital voice recorder.

In particularly preferred embodiments of the invention, the microphone is an electronic microphone that converts sound into an electrical signal. Such microphones are preferred due to being relatively inexpensive to manufacture and source when compared to other types of acoustic sensors. This makes it more cost effective to use with a larger group of cows at any one time. A multitude of technologies are available to measure acoustic signals, including but not limited to piezoelectric, laser, fibre optic, hydrophone, or a microelectrical-mechanical system.

In exemplary embodiments of the invention, the microphone includes a housing. Given its likely exposure to potentially harsh inclement weather when located on the dairy cow, it is preferable that the housing be waterproof, while being able to transmit sound to the microphone. Therefore, the housing may be configured from plastic or rubber materials. This is not meant to be limiting and other materials may be used.

The housing of the microphone may be configured to be integrated with an attachment means to facilitate the placement of the acoustic sensor on the dairy cow.

In exemplary embodiments of the present invention, the attachment means is a strap. The strap may include hook and loop material such as VELCRO^{™} or the like. For example, a portion of the housing may include hook material while the strap bears corresponding loop material. In this way, the strap allows the microphone to be attached to the rear legs of the cow with which it is to be used.

In some embodiments, the microphone may be configured with a strap that includes a pocket or sleeve into which the microphone is inserted. The strap then wraps around the pocket and leg of the cow and is held in place with VELCRO^{™}.

In other embodiments of the invention, the housing of the microphone may be configured with an attachment surface to which a bonding agent, such as high-strength adhesive, can be applied. Alternatively, the attachment surface may have a previously prepared adhesive layer which is activated prior to being attached to the cow by removing a shielding strip.

In these embodiments, the user attaching the microphone may need to prepare the area to which it is to be attached, by shaving it. This reduces the amount of hair at the desired location and allows the bonding of the housing and the microphone to be against at least a portion of the skin of the cow rather than its hair. This may result in a more durable attachment to the animal.

In some embodiments, the microphone may be placed in a waterproof plastic bag which is then taped to the leg of the animal. These examples of attaching the microphone to the cow are not meant to be limiting; persons skilled in the art will readily appreciate other ways of achieving the placement of the acoustic sensor to the animal.

The microphone should be understood to include a processor, such as a programmable logic controller (PLC) or processor.

The microphone should be understood to include a data storage device to collect audio signals over a period of time and store it for later retrieval and analysis. In exemplary embodiments of the present invention, the data storage device is a hard drive or memory device connected or otherwise linked to the processor.

In exemplary embodiments of the invention, the processor is configured to measure and record data relating to audio signals of specific intensity and/or spectral signature and the duration of these sounds. In such embodiments, the data will need to be extracted and later run through an algorithm for analysis by a central processing station or computer. This may be achieved by removing the hard drive or memory device or by directly connecting the acoustic sensor, once it has been removed from the cow, to the central processing station or computer.

In some embodiments of the invention, the processor is also configured to analyse the data relating to audio signals of specific intensity and/or spectral signature and the duration of said signals and correlate these with the criteria set out below defining a urination event and be identified as such.

In exemplary embodiments of the present invention, the microphone includes a power source in the form of a battery. For ease and cost efficiencies, lithium or button cell batteries are preferred but this is not meant to be limiting. The batteries may be larger (or smaller), depending on the size of the housing of the microphone and the required duration that the cow needs to be monitored. Smaller batteries are preferred since this allows the size of the overall housing to be kept to a minimum and thus potentially less obtrusive to the cow.

In alternative embodiments, as the cow likely is exposed to sunlight for several hours a day, a solar cell may be used as a source as power. This may be particularly useful if the measurements are to be performed during the summer months when there is a greater likelihood of extended periods of sunlight.

In some embodiments of the invention, the microphone may include means for wirelessly transmitting recorded audio signals, either in real time or at regular intervals, to a remote processing station for analysis. For a dairy cow, this might be performed as the cow moves along the race towards or exiting the milking shed. These locations, being routinely passed by the cow as they ambulate around the dairy farm, make for ideal placement of receivers for the transmitted recorded audio signals. However, it will be appreciated that any wireless transmissions of data may place additional demands on available power for the microphone.

The present invention calls for the use of one audio recording device. However, it is more preferable to use two audio recording devices, one for each rear leg of the animal. This provides extra information on urination events to improve the method for determining the time and duration of urination events.

Having two audio recording devices attached to the animal allows for the use of a triangulation procedure to determine the direction of the sound source relative to the two sensors. Cows urinate directly behind their rear legs and the time for the sound to travel to the two sensors are approximately equal. Urination events by nearby cows will be different distances to these two sensors and the travel times from the sound source to the two sensors will accordingly be different. The sound will reach the closer audio recording device before the other device.

A healthy dairy cow urinates on average around 12 times a day, although this is dependent on its diet. Each time a cow urinates should be understood to be a urination event. From observations, the inventors have found that in healthy dairy cows, at least 95% to 99% of urination events will have a duration of at least 5 seconds or more.

Urination events of duration less than 5 seconds are not expected to pose a nitrogen leaching risk. The nitrogen load from these shorter duration urination events less than 4 grams each.

Thus, according to one embodiment, the criteria for determining whether a urination event has occurred are:
1. an audio signal of a specific sound intensity;
2. an audio signal with a spectral signature of a specific spectral signature to within an error tolerance;
3. an audio signal meeting the criteria of 1. and 2. and having a duration of five seconds or more.

The majority (95 to 99%) of urination events are greater than five seconds in duration and less than 60 seconds in duration. Urination-like events of duration less than five seconds or greater than 60 seconds are therefore excluded.

As noted above, audio recordings reach the devices on the left and right leg at approximately the same time. Therefore, in embodiments where two audio recording devices are present, one for each leg, an additional criteria for detected audio signals to qualify as a valid urination event may be that:
4. the time difference between audio signals received by the audio recording devices must be less < 0.4 milliseconds, which represents approximately 25% of the spatial locations within two metres distance from the midpoint of the two audio recording devices.

Those skilled in the art will appreciate that the value of 0.4 millisecond is a tuning parameter that can be modified to provide the best performance of the algorithm for cows of different age, stature and breed.

The second recording device also provides a level of redundancy whereby criteria 1 to 3, or optionally 1 to 4, above must be satisfied from the second recording device for a urination event. These provide additional criteria that may be used for further accuracy.

In some embodiments of the invention, additional acoustic recording devices can be placed on the animal (for example, on the rump, upper or lower leg, or tail). Having a total of three or four recording devices to undertake an exact triangulation procedure based on travel times for the sound to reach each device to obtain the exact location of the sound source relative to the location of each acoustic recording device provides greater accuracy.

Alternatively, the audio recording device may be a stereo microphone that allows sound to be recorded from two different microphone locations. In preferred embodiments, where both of the rear legs of the animal are provided with acoustic recording devices, this means that four microphone locations may be present (two for each leg).

This is useful for establishing further criteria to improve the present method. For example, cows are always stationary when they urinate. Having three microphones attached to a cow can be used to better triangulate the urination-like sound and establish how the location of the sound source moves relative to the cow during period of the urination-like sound event. This provides an extra constraint to ensure that cows walking past other cows urinating are not associated with a urination event.

In addition, it should be appreciated that accelerometers and/or other reference audio signals can be used to determine periods of locomotion to identify if the cow is moving and/or the characteristic sound of a hoof striking the ground. Furthermore, individual urination events that are recorded by audio recording devices on multiple cows are associated to cows that record the event as the loudest. A total of three audio recording devices that allow triangulation of the urination event also allows the timecourse of the location where the urine strikes the ground during a urination event to be tracked, which displays a characteristic movement towards the hoofs of the cow toward the end of the event as the urine flow rate decreases.

The criteria set out above can be used to develop an algorithm to which audio signals, either in real time or in later analysis, is applied to determine the number of urination events that have occurred during the monitoring period and the average volume of urine excreted, and therefore the nitrogen load.

It will be appreciated that urination volume is proportional to the duration of the urination event, with an average flow rate of approximately 10 litres per minute. The nitrogen load of an individual event is also highly correlated with the time elapsed from the previous event, the time of day and the duration of the urination event.

The criteria established above calls for comparison of the spectral signature of the audio signal recorded by the acoustic sensor to a specific spectral signature that serves as a reference. It will be appreciated that the recorded sound intensity and spectral signature may depend partially on the substrate on which the cow urinates, such as concrete, soil, or pasture.

This reference spectral signature may be factored into the algorithm that analyses the audio signals to determine whether a urination event has occurred by comparing the similarity in the power spectral density to a reference power spectral density on each surface.

This means it is necessary to define a reference sound for each substrate, whether it be pasture, concrete (for example, in a milking shed), gravel (for example, raceways), grass or pasture (for example, paddocks) or dirt (for example, bare ground) to allow for the comparison to be made. Persons skilled in the art will appreciate that these are non-limiting examples, and the reference sounds may ultimately depend on the environment in which the animal is kept.

Descriptive statistics and features of an audio signal that may be used for comparative purposes against the audio signals recorded by the acoustic sensor include, but are not limited to the mean frequency, median frequency, maximum, minimum, cumulative maximum, cumulative minimum, moving median absolute deviation, root mean square level, distances between spectral peaks, mean power, occupied bandwidth, signal to noise ratio, signal to noise and distortion ratio, spectral entropy, spectral kurtosis, spectral flux, spectral flatness, spectral roll, spectral centroid, time-frequency complexity measure, Melfrequency cepstral coefficients, and the magnitude squared coherence. Furthermore, these descriptive statistics can be combined via a multiple regression, support vector machine, linear discriminant analysis, hidden Markov models, Gaussian mixture models, Random Forests, and artificial intelligence and machine learning methods such as neural networks, deep learning and image classification convolutional neural networks to classify sound signals as urination events.

Furthermore, the multidimensional spectra can be reduced in dimension using methods such as Principal Component Analysis, independent component analysis and singular value decomposition. The magnitude-squared coherence is a function of the power spectral densities and the cross power spectral density of two sound signals. The magnitude-squared coherence value is between 0 and 1 and indicates how well two sound signals correspond at each frequency. The cross power spectral density of two sound signals provides information on the power shared at a given frequency for the two signals.

Alternatively, the distance between the power spectral density of an unknown sound source (the audio signal detected by the audio recording device) and a reference known sound source (urination of pasture) can be compared using a variety of metrics. Such metrics include but are not limited to the Euclidian, Standardized Euclidian, Mahalanobis, City Block, Minkowski, Chebychev, Cosine, Correlation and customized distances. Such metrics may be applied to the raw power spectral density or appropriately transformed pre-processed power spectral density. Transformations include but are not limited to mean-centring and the standard normal variate.

Furthermore, the initial acoustic data may be pre-processed using a filter such as, but not limited to, a band-pass filter to remove the effects of wind, heavy rain and other noise. The error tolerance or required distance to the reference known sound source is a tuneable parameter and is dependent on the hardware, method of spectral analysis (including the sampling frequency, spectral leakage, time resolution, frequency resolution and frequency limits) and required sensitivity, specificity, precision, and accuracy of the method. The reference urine spectral signature, error tolerance to the reference known sound source, required sound intensity and five second threshold are also species-specific due to differences in urine flow rate, animal height and other factors.

An advantage of being able to classify and determine the substrate over which the urination event has occurred is that this is useful information for predicting the likely fate of the urine nitrogen in terms of nitrogen leaching into ground water and gaseous nitrogen emission to the atmosphere.

In use, the user will attach an audio recording device, which in exemplary embodiments of the invention is in the form of a microphone, to one of the rear legs of the cow to be monitored. Preferably, the microphones are attached to the medial metatarsus; this is relatively low down on the leg, close to ground level. This optimises capture of the acoustic signals associated with the urine stream contacting the ground during the urination event.

In preferred embodiments, for additional accuracy of the detection of a urination event, a further microphone may be attached to the other (second) rear leg of the cow. This allows the use of triangulation techniques to be employed when assessing the audio signals received during the urination event.

In addition, further audio recording devices or even an orientation sensor such as an accelerometer may be attached to the animal. Data from this accelerometer may be used to correlate and substantiate data derived from the audio signals, to provide additional information about the urination event. A non-limiting example of this may be whether the cow is stationary during a urination event.

Traditional microphones are omnidirectional and record sounds from all directions. The microphones may however be directional in nature, which can selectively record sound from a particular direction and target the likely direction of the sound from the urine stream striking the ground.

Once activated, the microphone records detected audio signals. The processor, whether as part of the microphone or a separate central processing station, processes these audio signals, to establish whether any of the detected signals meet the criteria for a urination event. Over time, this would build up a profile for the animal.

After a desired period of time, typically 72-hours, the user will retrieve the microphones from the cow. The collected data can be retrieved and processed through a central processing station, such as a computer. From this an average volume of urine excreted per urination event, urination time and daily urination frequency data can be determined for an individual cow.

The simplicity of attachment and removal of the microphone allows the user to more easily assess a group of cows. Furthermore, the non-invasive manner of attachment and removal is less stressful for the animals being assessed. Being relatively inexpensive, a stockpile of microphones may be built up and reused on a regular basis for groups or herds of cows.

Further aspects of the invention, which should be considered in all its novel aspects, will become apparent to those skilled in the art upon reading of the following description which provides at least one example of a practical application of the invention.

### Brief Description of the Drawings

One or more embodiments of the invention will be described below by way of example only, and without intending to be limiting, with reference to the following drawings, in which:
- Figure 1: is a schematic of a cow showing the placement of the acoustic sensors, in the form of audio recording devices, of the present invention;
- Figure 2: is a schematic diagram of the audio recording device of the present invention and attachment strap;
- Figure 3a: is a graph of the audio signal generated by a cow's first urination event over a certain period of time;
- Figure 3b: is a graph of the audio signal generated by a cow's second urination event over a certain period of time;
- Figure 4a: is a spectrogram of the audio signal generated by the first urination event of Figure 3a;
- Figure 4b: is a spectrogram of the audio signal generated by the second urination event of Figure 3b;
- Figure 5a: is a contour plot of the absolute time difference in milliseconds between recorded urination events at different spatial locations proximal to two audio recording devices located on the rear legs of the cow;
- Figure 5b: is a contour plot of the time difference in milliseconds between recorded urination events at different spatial locations proximal to two stereo microphones on the audio recording device located on the rear left and right legs of the cow respectively;
- Figure 6: is the mean power spectra for urination events that strike pasture;
- Figure 7: is a graph of the relationship between the measured and predicted duration of urination events for the 53 predicted urination events of a calibration trial following application of Model B; and
- Figure 8: is a graph of the relationship between the measured and predicted duration of urination events for the 53 predicted urination events of a calibration trial following application of Model D.

### Detailed Description of Preferred Embodiments of the Invention

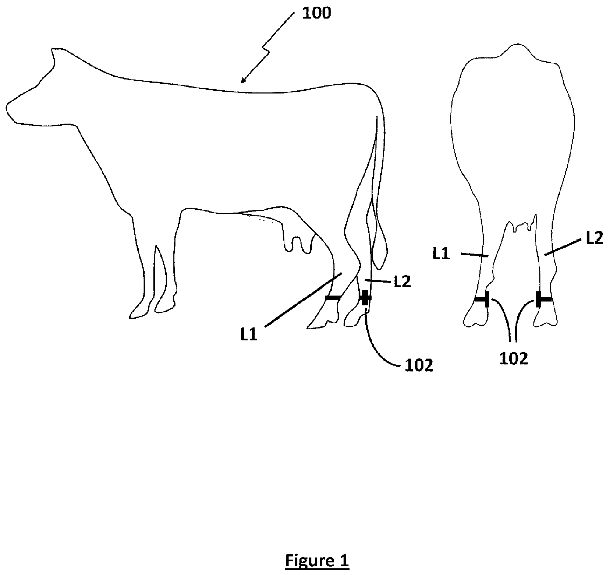

In Figure 1, a cow (generally indicated by arrow 100) is illustrated with the acoustic sensors, audio recording devices 102), in place. As can be seen, there are two sensors shown; one for each of the rear legs (L1, L2), positioned close to the hock. However, it will be appreciated that the invention may be performed with only one acoustic sensor or alternatively, two, three or four.

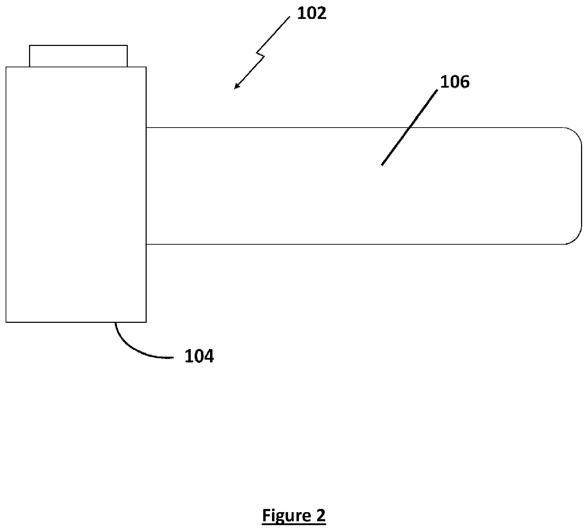

The audio recording devices (102), shown in Figure 2, may be standard, off-the-shelf, microphones such as an Olympus^{™} WS-853 digital voice recorder. This has a built in stereo microphone and the ability to record a variety of sampling frequencies (128 kbps 44.1 kHz; 64 kbps mono 44.1 kHz; 8 kbps mono 11.025 kHz). It is also relatively easy to source. However, it should be appreciated that the audio recording device may instead be a custom built solution which may be more cost effective.

The housing (104) of the audio recording device (102) has dimensions of 111.5 mm × 39 m × 18mm and weighs 77 grams (with its power source, two AAA batteries, inserted). The microphone is operative in temperatures ranging from 0 to 42 °C, has a recording time of 74-110 hours (8kbps mono) depending on battery type (Alkaline dry cell or Nickel-metal hydride rechargeable battery) and the ability to include extra memory (microSD card). This makes it ideal for use in the present invention.

To facilitate attachment of the audio recording device (102) to the cow, the housing (104) is provided with a strap (106). This is wrapped around the leg and back onto itself, secured with VELCRO^{™}. However, this is only one way of attaching the audio recording device and other methods will be readily envisaged by persons skilled in the art. For example, the audio recording device may be placed in a thin waterproof plastic bag which in turn is secured to the rear leg of the cow through the use of adhesive tape or the like.

Regardless of the method of securing the audio recording device to the cow, it is desirable that it remain in place for at least 48- to 72-hours. This is considered to be a sufficient period of time in which to monitor the number and duration of urination events and determine a reasonably accurate average for the cow. However, this is not meant to be limiting. In some cases, it may be acceptable to determine a urination frequency/duration average based on data collected over a 24-hour period or over a period greater than 72 hours depending on the desired accuracy.

Irrespective of the length of time that the audio recording device is in place, it must have a source of power for the processor that measures and records the audio signals. As mentioned above, in the described microphone this is in the form of two AAA batteries located within the audio recording device with two additional AA batteries but this is not meant to be limiting. Other types of microphones may require different batteries. The audio recording device also includes a hard drive or memory device to store the collected data for later retrieval and analysis.

As previously discussed, a urination event must meet three criteria in order to be identified as such. Thus, the criteria for determining whether a urination event has occurred are:
1. an audio signal with a sufficient sound intensity;
2. an audio signal with a spectral signature sufficiently close to a specific spectral signature to within an error tolerance;
3. an audio signal meeting the criteria of 1. and 2. and having a duration of five seconds or more.

An algorithm can be developed based on these parameters, and additional criteria as discussed previously in this specification, and applied by a processor to audio signal data collected from a cow fitted with audio recording devices.

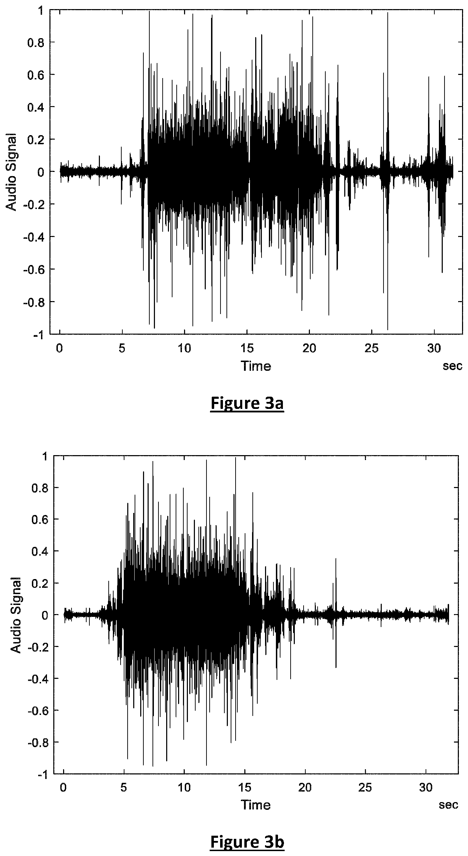

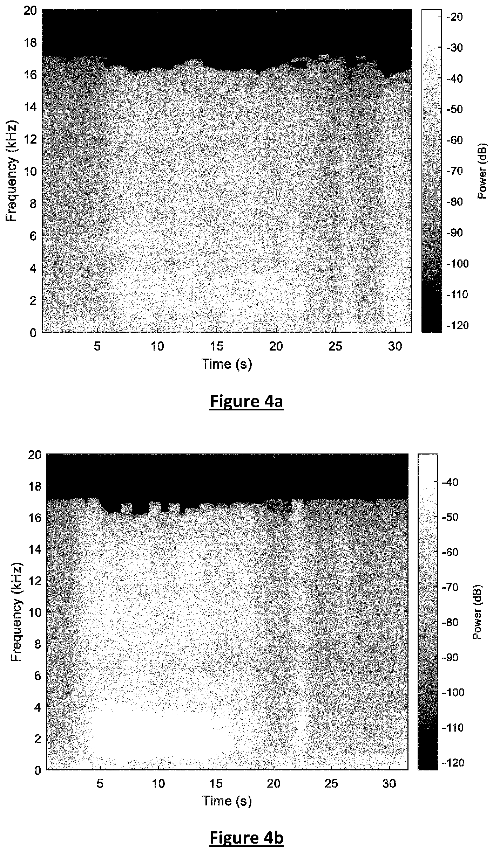

The implementation of the method, apparatus and system can be seen in Figures 3a, 3b, 4a and 4b; these respectively show the audio signals over a period of 30 seconds. It should be noted that the units in Figure 3a, 3b are normalized according to the MP3 file format, although persons skilled in the art will appreciate that the described methodology extends to any other digital sound format.

Figures 3a and 4a show a first urination event and Figures 3b and 4b illustrate a second urination event, both of which were visually assessed to have duration of 17 seconds, for a cow fitted with one acoustic sensor.

From this, it can be readily seen in Figure 2a that at approximately 7 seconds, a urination event occurred with a duration of 17 seconds according to criteria 1 to 3 above. This is consistent with the visual assessment.

However, small spikes in the audio signal were detected at 26 seconds and 31 seconds respectively. The amplitude of the spike at 26 seconds is not inconsistent with those of the urination event. However, its duration is less than five seconds and so it, along with the spike at 31 seconds, is too brief to be considered a urination event.

Furthermore, these small spikes can be eliminated as urination events when the data of Figure 3a is expressed as a spectrogram, as shown in Figure 4a. This expresses the power spectral density of the audio signals over the same period of time as Figure 3a. Those skilled in the art will appreciate that there are a multitude of spectrogram types and those shown in Figures 4a and 4b are classical spectrograms.

It will be seen that these small spikes have spectral profiles that differ from the urination event that occurred from 7 seconds to 23 seconds for the first event. This example shows the value in assessing more than one feature or property of the recorded sound for determining whether a urination event has occurred.

In Figures 3b and 4b, illustrating the detection of the second urination event. The urination event is shown to begin at about 4 seconds, and to end at about 19 seconds, with a duration of 15 seconds according to criteria 1 to 3 above, which is consistent with the visual observation of the event.

Audio recording devices can also be located proximal to the vulval folds to record the sound of the urine as it immediately exits the animal. However, the intensity of the sound is much lower than that as the urine strikes the ground hence the preference for the placement of the audio recording devices low on the rear legs of the animal.

The sound intensity of the urination event is dependent on the urination flow rate and also the distance from the point where the urine strikes the ground to the audio recording device(s). During a urination event the urine flow velocity increases to a maximum and then decreases during the later stage of the urination event. This is associated with a characteristic increase in the sound intensity to a maximum followed by a decrease during the later stages of the urination event.

This is demonstrated in Figures 3a and 3b. The conclusion of the urination event is normally followed by one to four very short low volume spurts of urine that clear the urinary tract. These are referred to as micro urination events. These very short urination bursts provide another criteria to classify events, whereby each sustained urination event is followed by at least one micro event. This provides a means to distinguish true urination events from nearby water flow, such as from hoses or drainpipes of any buildings in close proximity to the cow.

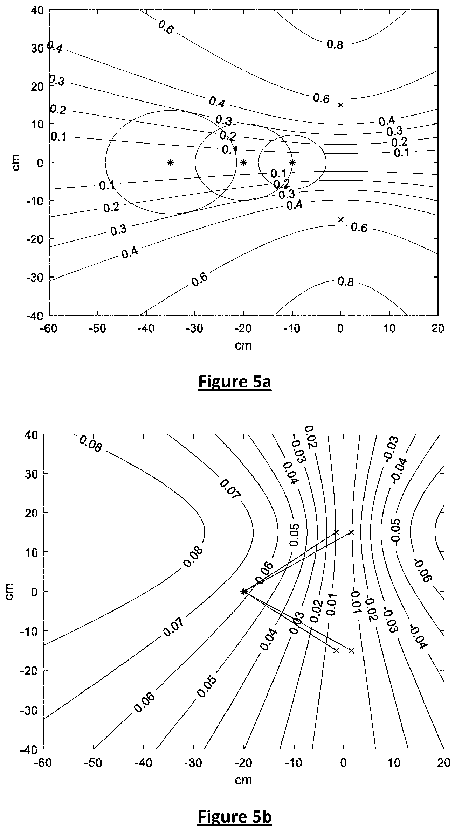

Turning now to Figure 5a, this depicts a contour plot of the absolute time difference in milliseconds between recorded urination events at different spatial locations proximal to the preferred embodiment of the invention which utilises two audio recording devices located on the rear legs of the cow. The use of two audio recording devices allows for the use of triangulation methods for greater accuracy of detecting audio signals and then correlating them to a urination event.

This is based on the speed of sound in air of 331 metres per second at 0°C. It should be appreciated that adjustments to allow for the effect of temperature on the speed of sound can be readily made, e.g. at temperatures of 30 °C, the speed of sound is 349 meters per second.

The location of the two audio recording devices on the rear legs of the cow, generally located to the right side of the graph, are denoted by crosses. Typical locations where the urine strikes the ground are shown by asterisks for different times of the urination event. The three circles denote the typical zones where urine strikes the ground for low medium and high urine flow rates during an individual event.

To qualify as a valid urination event, there must be a time difference of less than 0.4 milliseconds between the audio signals received by the microphones. This reduces or eliminates the possibility that the microphones detect a urination event from a nearby cow and which is detected by the audio recording devices.

Other techniques may be employed to improve accuracy of the method and reduce or eliminate false positives that could be inadvertently assessed as a urination event for the cow. For example, audio recording devices incorporating a pair of stereo microphones may be used.

An example of a potential false positive may be a dung event. However, the criteria outlined above may disqualify audio signals arising from a dung event. These typically have distinct gaps in the signal rather than being a continuous signal, as with a urination event, and thus do not have a continuous duration of five seconds or more.

Figure 5b is a contour plot of the time difference in milliseconds between recorded urination events at different spatial locations proximal to two stereo microphones on the audio recording device located on the rear left and right legs of the cow respectively. The numbers on the contour lines denote the difference in milliseconds between the time for the sound to reach the microphone farther from the sound source, i.e. the sound created as the urine stream contacts the ground, and the stereo microphone closer to the sound source.

On each audio recording device, the individual stereo microphones are spaced a distance of three centimetres apart. The location of the four microphones on the two audio recording devices on the left and right rear legs of the cow are denoted by crosses. As with the previous figure, the cow is generally located on the right side of this figure.

Typical locations where the urine strikes the ground are shown by an asterisk for a particular time during a urination event. Triangulation can be employed to uniquely determine the location of the point where the urine strikes the ground. In this example, the urine stream contacts the ground a distance of approximately 20 centimetres behind the centreline of the two audio recording devices.

It will be appreciated that the sound intensity is proportionally greater for the microphone closer to the sound source. In this case, the sound of the impact of the urine stream contacting the ground reaches the microphones closer to the sound source 0.062 milliseconds before the microphones farther from the sound source. This is based on the speed of sound in air of 331 meters per second at 0°C, which as previously noted, can be adjusted to allow for the effect of temperature on speed of sound.

As can be seen in Figure 5b, the spatial location where the urination event strikes the ground is equidistant from the two audio recording devices and therefore each device records the event at the same time. This provides greater accuracy in determining that a urination event has occurred for that particular cow, rather than an animal located nearby.

It will be appreciated that the audio recording devices must have synchronised clocks achieved by a formal procedure (e.g. by attaching or communicating with a computer device) or by producing a calibration sound (with a characteristic spectral profile) at a specified distance from each acoustic sensor. Alternatively, in-field on-farm audio recordings can be analysed to maximise the crosscorrelation between multiple audio recordings to estimate the time differences between the clocks on these devices.

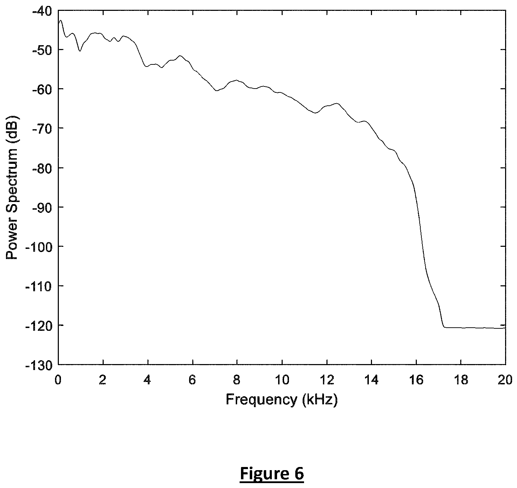

Figure 6 shows a representation of the mean power spectra for urination events that strike pasture, which serves as a reference. As part of the process for assessing whether a urination event has occurred, the power spectra of the audio signal received by the audio recording devices is compared against the reference signal. If the comparison can be made within a margin of error, then it can be satisfied that a urination event has occurred (subject to being of appropriate duration and any other applicable criteria).

If the comparison cannot be made, then the power spectra of the audio signal is compared against a reference power spectra for urination events that strike concrete. Again, if the comparison can be made within a margin of error, then it can be satisfied that a urination event has occurred. If not, the comparisons can be made against remaining reference power spectra for urination events occurring on gravel, bare dirt, bedding and so on.

Further implementation of the method in respect of a group of dairy cows shall now be described. A trial was conducted with 15 cows over a 4-day period. For this trial, each cow was provided with an Olympus^{™} WS-853 digital voice recorder, placed in a waterproof plastic bag and attached to a rear leg on the first day of the trial and removed at the end of the last day of the trial. Environmental conditions during the trial were generally windy, particularly on the third and fourth day. The third day included periods of rain, including a thunderstorm at around 17:00.

Cows were visually assessed for time and duration of urination events over a 6-hour period (09:00 to 15:00). A total of 145 urination events were observed over this six-hour period with one further urination event observed at exactly 15:00. Audio recordings were also used to independently assess the time and duration of events. The duration of events was defined as the sum of periods of urination during and event (i.e. pauses during the urination process were excluded).

Dung events in the trial were found to have relatively high moisture content than typical and these dung events were audible as multiple events separated by ^{~}0.25 second delay between events.

Urination events are characterised by acoustic signals with a spectral profile and sound power that are typical of most urination events. Urination events are also typically greater than 4 seconds in duration. These observations were used to develop a range of models to classify urination events, the event time and predict event duration.

For calibration purposes, 61 urination events and 443 non-urination events were obtained from the audio recordings outside the visual observation period (obtained 17:00 to 19:00 on days 1,2,3 and 06:30 to 08:30 on Day 4 of the trial for a total of 120 hours of audio).

The power spectrum was calculated with a 0.04 second time resolution. It was necessary to multiply the power by 3.0 to adjust for the effect on the acoustic signal of the waterproof plastic bag that housed the audio recording devices used in the trials. Models were developed based on 44 selected spectral features of a candidate urination event. The instantaneous frequency (IF), signal power (P; calculated from frequency (F) 1 kHz to 16 kHz) and the minimum Euclidian distance to ten reference power spectra (E) were then calculated every 0.01 second. The ten reference power spectra were filtered (the power was set to zero for frequencies less than 1 kHz to remove the effects of environmental wind noise) and then preprocessed using a Standard Normal Variate (SNV) transformation. The ten reference spectra were obtained from a representative urination event outside the observation period (this reference event was uncontaminated by environmental noise).

The 44 selected spectral features of a candidate urination event are:
1. The maximum power P;
2. The number of seconds the power P is greater than 0.1;
3. The number of seconds the power P is greater than 0.2;
4. The number of seconds the power P is greater than 0.35;
5. The number of seconds the power P is greater than 0.5;
6. The maximum time interval where the power P is greater than 0.2;
7. The maximum time interval where the power P is greater than 0.1;
8. The standard deviation in the power P;
9. The standard deviation in the power P greater than 0.2;
10. The standard deviation in the power P greater than 0.35;
11. The standard deviation in the power P greater than 0.5;
12. The maximum time interval where the power P is greater than 0.3;
13. The minimum Euclidian distance to the reference spectra (E);
14. The number of seconds the Euclidian distance to the reference spectra (E) is less than 37;
15. The number of seconds the Euclidian distance to the reference spectra (E) is less than 32;
16. The number of seconds the Euclidian distance to the reference spectra (E) is less than 27;
17. The number of seconds the Euclidian distance to the reference spectra (E) is less than 20;
18. The maximum time interval where the Euclidian distance to the reference spectra (E) is less than 32;
19. The maximum time interval where the Euclidian distance to the reference spectra (E) is less than 37;
20. The standard deviation in the Euclidian distance to the reference spectra (E);
21. The standard deviation in the Euclidian distance to the reference spectra (E) less than 37;
22. The standard deviation in the Euclidian distance to the reference spectra (E) less than 32;
23. The standard deviation in the Euclidian distance to the reference spectra (E) less than 27;
24. The maximum time interval where the Euclidian distance to the reference spectra (E) is less than 27;
25. The number of seconds that P×(45-E) is greater than zero;
26. The maximum time interval where the Euclidian distance to the reference spectra (E) is less than 24;
27. The maximum time interval where the power P is greater than 0.4;
28. The maximum time interval where P×(45-E) is greater than 7;
29. the number of seconds in the candidate event (where P>0.1 and E<37);
30. the number of seconds in the candidate event (where P>0.1 and E<37) (acoustic 0.5 second gaps were filled with 50 0.01 second dilations followed by 50 0.01 second erosions);
31. The maximum time interval where the instantaneous frequency (IF) is greater than 2 kHz;
32. The proportion of the signal power in the frequency range 1000≤F≤1477;
33. The proportion of the signal power in the frequency range 1477≤F≤2069;
34. The proportion of the signal power in the frequency range 2069≤F≤2801;
35. The proportion of the signal power in the frequency range 2801≤F≤3708;
36. The proportion of the signal power in the frequency range 3708≤F≤4831;
37. The proportion of the signal power in the frequency range 4831≤F≤6223;
38. The proportion of the signal power in the frequency range 6223≤F≤7946;
39. The proportion of the signal power in the frequency range 7946≤F≤10081;
40. The proportion of the signal power in the frequency range 10081≤F≤12725;
41. The proportion of the signal power in the frequency range 12725≤F≤16000;
42. The number of crossing per seconds in the power P across 0.1;
43. The number of crossing per seconds in the Euclidian distance to the reference spectra (E) across 27;
44. The number of crossing per seconds in the instantaneous frequency (IF) across 1kHz.

Candidate urination event regions were calculated from time intervals (I₀) when P>0.1, E<37 and IF>1500Hz. This region was transformed via an erosion by 0.2 seconds, followed by a dilation of 0.4 seconds, followed by an erosion of 0.2 seconds (I₁). Regions that were less than 2 seconds from each other were then joined together via labelling the region I₁ with a 2 second dilation and a 2 second erosion (I₂). The region I₂ was then labelled and used to label the candidate urination event regions in I₁.

Models were calibrated to the urination events occurring outside the visual observation period. Seven models were also used to provide a consensus (MV) urination event model and a candidate event (CEV) model. In this example, the collective models, all based on selected spectral features of a potential urination event are:
Model 1 - Linear Discriminant Analysis (LDA) classification;
Model 2 - Naïve Bayes (NB) classification;
Model 3 - Support Vector Machine (SVM) classification;
Model 4 - Stepwise generalised linear model (SGLM) classification;
Model 5 - Classification Tree (CT);
Model 6 - Classification Ensemble (CE);
Model 7- k-nearest neighbour (kNN) classification;
Model 8 - at least one of Models 1-3,5-7 predicts a urination event (Candidate event vote (CEV));
Model 9 - at least four of Models 1-7 predicts an event (Majority vote (MV)).

It has been discussed that the substrate over which the urination event takes place may have a bearing on the properties of the audio signal. As an example, to differentiate between urination events that occur on pasture and concrete respectively, a set of 146 urination events were classified into 73 events on pasture and 73 events on concrete. This was based on expert assessment of a subset of the audio recordings (and time of recording which provided information about when cows were in yards) from another trial involving 50 cows over a five-day period.

These events were then used to develop a classification algorithm for urination surface type (pasture or concrete. 10 events were randomly selected to obtain reference spectra for each respective surface type (reference sounds were on average 7 seconds in duration). A further 30 events were used model calibration and the remaining 33 events were used for validation. Sounds were pre-processed as per the event classification models. Surface models were based a set of 20 spectral features:
1. The mean power P;
2. The standard deviation in the power P;
3. The mean minimum Euclidian distance to the concrete reference spectra (E_{c});
4. The standard deviation in the minimum Euclidian distance to the concrete reference spectra (E_{c});
5. The mean minimum Euclidian distance to the pasture reference spectra (Eₚ);
6. The standard deviation in the minimum Euclidian distance to the pasture reference spectra (Eₚ);
7. The mean instantaneous frequency (IF);
8. The standard deviation in the instantaneous frequency (IF);
9. The proportion of the signal power in the frequency range 1000≤F≤1477;
10. The proportion of the signal power in the frequency range 1477≤F≤2069;
11. The proportion of the signal power in the frequency range 2069≤F≤2801;
12. The proportion of the signal power in the frequency range 2801≤F≤3708;
13. The proportion of the signal power in the frequency range 3708≤F≤4831;
14. The proportion of the signal power in the frequency range 4831≤F≤6223;
15. The proportion of the signal power in the frequency range 6223≤F≤7946;
16. The proportion of the signal power in the frequency range 7946≤F≤10081;
17. The proportion of the signal power in the frequency range 10081≤F≤12725;
18. The proportion of the signal power in the frequency range 12725≤F≤16000;
19. The mean spectral entropy (F >1 kHz);
20. The standard deviation in spectral entropy (F >1 kHz).

A consensus (MV) urination surface model was developed as per the urination event model previously described.

The power spectra of the audio signals are distinctly different between urination events on pasture and concrete. Events on concrete were typically louder and had larger instantaneous frequency. Events on pasture tended to have a more stable sound on a 0.05 second timescale. Tables 1 and 2 show the comparison of observed and predicted classification of urination surface (concrete, pasture) in the trials, with Model 9 (MV Calibration). In these tables, TP - True Positive, FN - False Negative, FP - False Positive, TN - True negative, while the F1 score is the harmonic mean of Sensitivity and Precision and provides a balanced measure of binary classification.

**Table 1**

| **Model 9 (MV Calibration)** | **Predicted Concrete** | **Predicted Pasture** | |
|---|---|---|---|
| Measured Concrete | 28 (TN) | 2 (FP) | Precision 0.93±0.05 |
| | | | Sensitivity 0.90±0.06 |
| Measured Pasture | 3 (FN) | 27 (TP) | Specificity 0.93±0.05 |
| | | | F1 = 0.92 |

**Table 2**

| **Model 9 (MV Calibration)** | **Predicted Concrete** | **Predicted Pasture** | |
|---|---|---|---|
| Measured Concrete | 30 (TN) | 3 (FP) | Precision 0.92±0.05 |
| | | | Sensitivity 0.94±0.04 |
| Measured Pasture | 2 (FN) | 31 (TP) | Specificity 0.91±0.05 |
| | | | F1 = 0.93 |

Further models were developed for predicting the duration of the urination events.
Model A: The duration of the sufficient sustained acoustic signal was used as a predictor of the duration of the urination event;
Model B: The duration of the sufficient sustained acoustic signal was used as a predictor of the duration of the urination event (with gaps in the urination signal of up to 0.5 s filled);
   Spectral features of the urination acoustic signal were used as training data for Models C and D below.
Model C: Stepwise linear regression (SLR);
Model D: Support vector machine regression (SVMR).

Table 3 below shows the comparison of observed and predicted classification of urination events of the trial involving 15 cows, with Model 9 (MV Calibration).

**Table 3**

| **Model 9 (MV Calibration)** | **Unpredicted Urination Event** | **Predicted Urination Event** | |
|---|---|---|---|
| Unobserved Urination event | 442 (TN) | 1 (FP) | Precision 0.98±0.02 |
| | | | Sensitivity 0.87±0.04 |
| Measured Urination Event | 8¹ (FN) | 53 (TP) | Specificity 0.99±0.002 |
| | | | F1 = 0.92 |

| | | | |
|---|---|---|---|
| ¹ The duration of the 8 unpredicted events was not significantly different to the 53 predicted events. | | | |

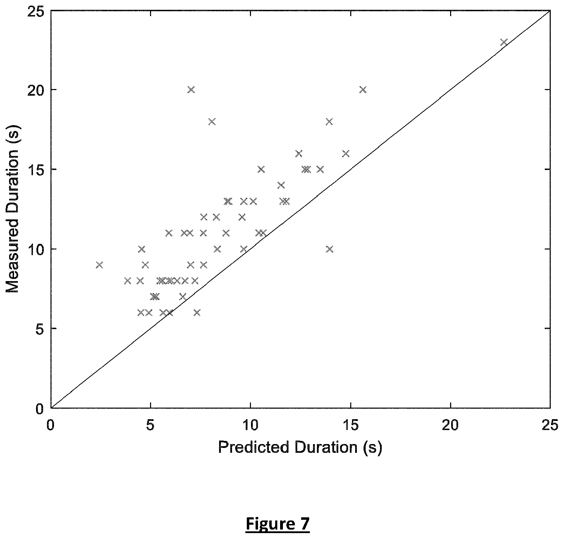

A graphical representation of the implementation of Model B is represented in Figure 7. This shows the relationship between the measured and predicted duration of urine events for the 53 SVM model predicted urination events in the trial of 15 cows (outside visual observation period) with Model B (R² = 0.61, RMSE = 2.5 seconds, N = 53, P < 0.001, slope = 0.84 ± 0.10, intercept = 4.0 ± 0.87 seconds, bias = - 2.65 seconds).

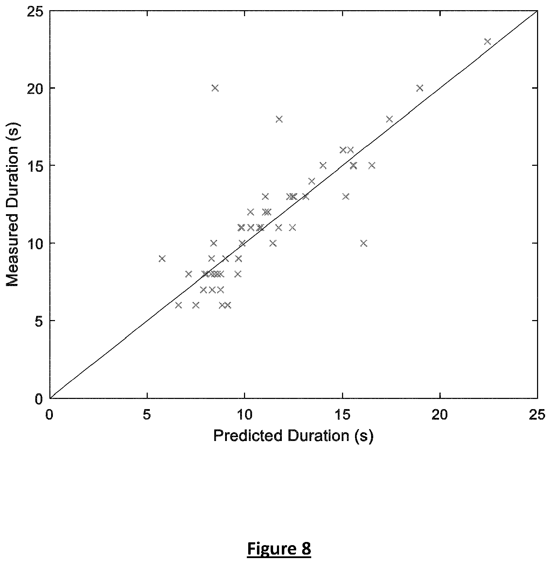

Model B slightly underpredicts event duration due to a delayed predicted start to an event and an early predicted event finish time (the sound intensity is low at these times and there are often urination dribbles at the start and end of an event). In contrast, Model D provides a more accurate prediction for urination event, as shown in Figure 8, which represents the relationship between the measured and predicted duration of urine events for the 53 SVM predicted urination events outside visual observation period with Model D (SVMR) (R² = 0.65, RMSE = 2.39 seconds, N = 53, P < 0.001, slope = 0.94 ± 0.10, intercept = 0.76 ± 1.13 seconds, bias = -0.11 seconds).

The present invention provides the means to determine whether a urination event has occurred for a particular animal, and the duration of that event.

Determination of the average amount of urine excreted per urination event for a particular cow can then be used to help make management decisions in relation to that cow. For example, cows that urinate more frequently may be preferentially selected for breeding (or their sires preferentially used for breeding) as such animals may have a lower impact on nitrogen loading on pasture. Alternatively, they may be retained in the herd while cows that urinate less frequently are culled or separated from the herd into groups that would graze pasture that is remote from water ways or poses a lower nitrogen leaching risk.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

Reference to any prior art in this specification is not, and should not be taken as, an acknowledgement or any form of suggestion that that prior art forms part of the common general knowledge in the field of endeavour in any country in the world.

Where in the foregoing description reference has been made to integers or components having known equivalents thereof, those integers are herein incorporated as if individually set forth.

It should be noted that various changes and modifications to the presently preferred embodiments described herein will be apparent to those skilled in the art. Such changes and modifications may be made without departing from the scope of the invention and without diminishing its attendant advantages. It is therefore intended that such changes and modifications be included within the present invention as defined in the appended claims.

## Claims

1. A method of detecting a urination event for a livestock animal (100), wherein the livestock animal has two rear legs, the method including the steps of:
a. placing an acoustic sensor (102) on a rear leg (L1);
b. monitoring for an audio signal of specific sound intensity and/or spectral signature;
c. measuring the duration of said audio signal; and
d. determining whether the duration of said audio signal corresponds to a urination event.

2. The method as claimed in claim 1, including the additional step of:
e. correlating the audio signal of specific intensity and/or spectral signature and the duration of said signals to a set of criteria defining a urination event to determine whether a urination event has occurred for the livestock animal.

3. The method as claimed in either claim 1 or claim 2, wherein the criteria for determining whether a urination event has occurred are:
a. an audio signal of a specific sound intensity;
b. an audio signal with a spectral signature of a specific spectral signature to within an error tolerance;
c. an audio signal meeting the criteria of a. and b. and having a duration of five seconds or more.

4. The method as claimed in claim 3, wherein the specific spectral signature is the sound intensity and spectral signature of an audio signal corresponding to the sound of urine contacting a substrate.

5. The method as claimed in any one of claims 1 to 4, wherein the method includes an additional step of placing a further acoustic sensor (102) on another rear leg of the livestock animal, and optionally the method includes an additional step of placing additional acoustic sensors on one or more of the following areas of the animal:
• the rump;
• tail;
• upper leg;
• lower leg.

6. The method as claimed in claim 5, wherein an additional criteria for determining whether a urination event has occurred is:
d. the time difference between audio signals received by the acoustic sensor and the further acoustic sensor (102) must be less < 0.4 milliseconds.

7. The method as claimed in any one of claims 1 to 6, wherein the livestock animal is a dairy cow.

8. An apparatus suitable for using the method of detecting a urination event for a livestock animal (100) according to any one of claims 1 to 7, and wherein the apparatus includes:
an acoustic sensor (102), wherein the sensor is configured to be placed on a rear leg (L1) of a livestock animal, and wherein the sensor includes:
a data collection device; and
a processor, wherein the processor is configured to record data relating to an audio signal of specific sound intensity, spectral signature, and duration.

9. The apparatus as claimed in claim 8, wherein the acoustic sensor (102) measures:
• sound intensity and/or
• sound pressure level measured in Pascals; and/or
• sound power level measured in Watts; and/or
• sound pressure level measured in decibels; and/or
• sound power level measured in decibels; and/or
• frequency measured in Hertz.

10. The apparatus as claimed in either claim 8 or claim 9, wherein the acoustic sensor (102) is:
• an audio recording device in the form of a microphone; or
• audio recording device in the form of a digital voice recorder.

11. The apparatus as claimed in claim 10, wherein the microphone is a stereo microphone configured to record sound from two different microphone locations.

12. The apparatus as claimed in any one of claims 8 to 11, wherein the acoustic sensor (102) includes a housing (104), wherein the housing of the acoustic sensor is configured:
• to be integrated with an attachment means (106); or
• with an attachment surface.

13. The apparatus as claimed in any one of claims 8 to 12, wherein the data storage device is a hard drive or memory device connected or otherwise linked to the processor.

14. The apparatus as claimed in any one of claims 8 to 13, wherein the apparatus includes:
• a power source in the form of a battery; and/or
• means wirelessly transmitting recorded audio signals.

15. A system suitable for using the method of detecting a urination event for a livestock animal (100) according to claim 2, and wherein the system includes:
an apparatus as claimed in any of claims 8 to 14; and
a processor configured to correlate audio signals of specific sound intensity, spectral signature, and duration to criteria defining a urination event and determine whether a urination event has occurred for said livestock animal.

16. The system as claimed in claim 15, wherein the processor is a central processing station.

## Patentansprüche

1. Verfahren zur Erkennung eines Urinierereignisses für ein Nutztier (100), wobei das Nutztier zwei Hinterbeine aufweist, wobei das Verfahren die Schritte beinhaltet:
a. Platzieren eines akustischen Sensors (102) an einem Hinterbein (L1),
b. Überwachen auf ein Audiosignal spezifischer Schallintensität und/oder spektraler Signatur;
c. Messen der Dauer des Audiosignals; und
d. Bestimmen, ob die Dauer des Audiosignals einem Urinierereignis entspricht.

2. Verfahren nach Anspruch 1, beinhaltend den zusätzlichen Schritt:
e. Korrelieren des Audiosignals spezifischer Intensität und/oder spektraler Signatur und der Dauer der Signale mit einem Satz von Kriterien, die ein Urinierereignis definieren, um zu bestimmen, ob bei dem Nutztier ein Urinierereignis stattgefunden hat.

3. Verfahren nach Anspruch 1 oder Anspruch 2, wobei die Kriterien zum Bestimmen, ob ein Urinierereignis stattgefunden hat, Folgende sind:
a. ein Audiosignal spezifischer Schallintensität,
b. ein Audiosignal mit einer spektralen Signatur einer spezifischen spektralen Signatur innerhalb einer Fehlertoleranz;
c. ein Audiosignal, das die Kriterien von a. und b. erfüllt und eine Dauer von fünf Sekunden oder mehr aufweist.

4. Verfahren nach Anspruch 3, wobei die spezifische spektrale Signatur die Schallintensität und spektrale Signatur eines Audiosignals ist, das dem Geräusch von Urin entspricht, der mit einem Substrat in Kontakt kommt.

5. Verfahren nach einem der Ansprüche 1 bis 4, wobei das Verfahren einen zusätzlichen Schritt des Platzierens eines weiteren akustischen Sensors (102) an einem anderen Hinterbein des Nutztiers beinhaltet, und optional wobei das Verfahren einen zusätzlichen Schritt des Platzierens zusätzlicher akustischer Sensoren an einem oder mehreren der folgenden Bereiche des Tiers beinhaltet:
• das Hinterteil;
• Schwanz;
• Oberschenkel;
• Unterschenkel.

6. Verfahren nach Anspruch 5, wobei ein zusätzliches Kriterium zur Bestimmung, ob ein Urinierereignis stattgefunden hat, Folgender ist:
d. der Zeitunterschied zwischen den vom akustischen Sensor und dem weiteren akustischen Sensor (102) empfangenen Audiosignalen muss geringer kleiner als < 0,4 Millisekunden sein.

7. Verfahren nach einem der Ansprüche 1 bis 6, wobei das Nutztier eine Milchkuh ist.

8. Vorrichtung, die für die Verwendung des Verfahrens zur Erkennung eines Urinierereignisses für ein Nutztier (100) nach einem der Ansprüche 1 bis 7 geeignet ist, und wobei die Vorrichtung beinhaltet:
einen akustischen Sensor (102), wobei der Sensor so konfiguriert ist, dass er an einem Hinterbein (L1) eines Nutztiers platziert wird, und wobei der Sensor beinhaltet:
ein Datenerfassungsgerät; und
ein Prozessor, wobei der Prozessor so konfiguriert ist, dass er Daten in Bezug auf ein Audiosignal mit spezifischer Schallintensität, spektraler Signatur und Dauer aufzeichnet.

9. Vorrichtung nach Anspruch 8, wobei der akustische Sensor (102) misst:
• Schallintensität und/oder
• Schalldruckpegel gemessen in Pascal; und/oder
• Schallleistungspegel gemessen in Watt; und/oder
• Schalldruckpegel gemessen in Dezibel; und/oder
• Schallleistungspegel gemessen in Dezibel; und/oder
• Frequenz gemessen in Hertz.

10. Vorrichtung nach Anspruch 8 oder Anspruch 9, wobei der akustische Sensor (102) Folgendes ist:
• ein Audioaufzeichnungsgerät in Form eines Mikrofons; oder
• Audioaufzeichnungsgerät in Form eines digitalen Stimmenrekorders.

11. Vorrichtung nach Anspruch 10, wobei das Mikrofon ein Stereomikrofon ist, das so konfiguriert ist, dass es Schall aus zwei verschiedenen Mikrofonpositionen aufzeichnet.

12. Vorrichtung nach einem der Ansprüche 8 bis 11, wobei der akustische Sensor (102) ein Gehäuse (104) beinhaltet, wobei das Gehäuse des akustischen Sensors konfiguriert ist:
• um mit einem Befestigungsmittel (106) integriert zu sein; oder
• mit Befestigungsfläche.

13. Vorrichtung nach einem der Ansprüche 8 bis 12, wobei das Datenspeichergerät eine Festplatte oder ein Speichergerät ist, die oder das an den Prozessor angeschlossen oder anderweitig mit diesem verknüpft ist.

14. Vorrichtung nach einem der Ansprüche 8 bis 13, wobei die Vorrichtung beinhaltet:
• eine Stromquelle in Form einer Batterie; und/oder
• Mittel, die aufgezeichnete Audiosignale drahtlos übertragen.

15. System, das für die Verwendung des Verfahrens zur Erkennung eines Urinierereignisses für ein Nutztier (100) nach Anspruch 2 geeignet ist, und wobei die System beinhaltet:
eine Vorrichtung nach einem der Ansprüche 8 bis 14; und
einen Prozessor, der so konfiguriert ist, dass er Audiosignale spezifischer Schallintensität, spektraler Signatur und Dauer mit Kriterien korreliert, die ein Urinierereignis definieren, und bestimmt, ob bei dem Nutztier ein Urinierereignis stattgefunden hat.

16. System nach Anspruch 15, wobei der Prozessor eine zentrale Verarbeitungsstation ist.

## Revendications

1. Procédé de détection d'un événement de miction pour un animal d'élevage (100), ledit animal d'élevage possédant deux pattes arrière, le procédé comprenant les étapes de :
a. placement d'un capteur acoustique (102) sur une patte arrière (L1) ;
b. surveillance d'un signal audio d'intensité acoustique et/ou de signature spectrale spécifiques ;
c. mesure de la durée dudit signal audio ; et
d. détermination pour savoir si la durée dudit signal audio correspond à un événement de miction.

2. Procédé selon la revendication 1, comprenant l'étape supplémentaire de :
e. corrélation du signal audio d'intensité et/ou de signature spectrale spécifiques et la durée desdits signaux à un ensemble de critères définissant un événement de miction pour déterminer si un événement de miction s'est produit pour l'animal d'élevage.

3. Procédé selon la revendication 1 ou la revendication 2, lesdits critères pour déterminer si un événement de miction s'est produit étant :
a. un signal audio d'une intensité acoustique spécifique ;
b. un signal audio avec une signature spectrale d'une signature spectrale spécifique dans une tolérance d'erreur ;
c. un signal audio répondant aux critères de a. et b. et possédant une durée supérieure ou égale à cinq secondes.

4. Procédé selon la revendication 3, ladite signature spectrale spécifique étant l'intensité acoustique et la signature spectrale d'un signal audio correspondant au son de l'urine entrant en contact avec un substrat.

5. Procédé selon l'une quelconque des revendications 1 à 4, ledit procédé comprenant une étape supplémentaire de placement d'un capteur acoustique supplémentaire (102) sur une autre patte arrière de l'animal d'élevage, et éventuellement ledit procédé comprenant une étape supplémentaire de placement de capteurs acoustiques supplémentaires sur une ou plusieurs des zones suivantes de l'animal :
• la croupe ;
• la queue ;
• la partie supérieure de la jambe ;
• la partie inférieure de la jambe.

6. Procédé selon la revendication 5, un critère supplémentaire pour déterminer si un événement de miction s'est produit étant :
d. la différence de temps entre les signaux audio reçus par le capteur acoustique et l'autre capteur acoustique (102) doit être inférieure à < 0,4 millisecondes.

7. Procédé selon l'une quelconque des revendications 1 à 6, ledit animal d'élevage étant une vache laitière.

8. Appareil adapté à l'utilisation du procédé de détection d'un événement de miction pour un animal d'élevage (100) selon l'une quelconque des revendications 1 à 7, et ledit appareil comprenant :
un capteur acoustique (102), ledit capteur étant configuré pour être placé sur une patte arrière (L1) d'un animal d'élevage, et ledit capteur comprenant :
un dispositif de collecte de données ; et
un processeur, ledit processeur étant configuré pour enregistrer des données relatives à un signal audio d'intensité acoustique, de signature spectrale et de durée spécifiques.

9. Appareil selon la revendication 8, ledit capteur acoustique (102) mesurant :
• une intensité acoustique et/ou
• un niveau de pression acoustique mesuré en Pascals ; et/ou
• un niveau de puissance acoustique mesuré en watts ; et/ou
• un niveau de pression acoustique mesuré en décibels ; et/ou
• un niveau de puissance acoustique mesuré en décibels ; et/ou
• un fréquence mesurée en Hertz.

10. Appareil selon la revendication 8 ou la revendication 9, ledit capteur acoustique (102) étant :
• un dispositif d'enregistrement audio sous la forme d'un microphone ; ou
• dispositif d'enregistrement audio sous la forme d'un enregistreur vocal numérique.

11. Appareil selon la revendication 10, ledit microphone étant un microphone stéréo configuré pour enregistrer le son provenant de deux emplacements de microphone différents.

12. Appareil selon l'une quelconque des revendications 8 à 11, ledit capteur acoustique (102) comprenant un boîtier (104), ledit boîtier du capteur acoustique étant configuré pour :
• être intégré à un moyen de fixation (106) ; ou
• à une surface de fixation.

13. Appareil selon l'une quelconque des revendications 8 à 12, ledit dispositif de stockage de données étant un disque dur ou un dispositif de mémoire connecté ou autrement lié au processeur.

14. Appareil selon l'une quelconque des revendications 8 à 13, ledit appareil comprenant :
• une source d'alimentation sous la forme d'une batterie ; et/ou
• un moyen transmettant sans fil des signaux audio enregistrés.

15. Système adapté à l'utilisation du procédé de détection d'un événement de miction pour un animal d'élevage (100) selon la revendication 2, et ledit système comprenant :
un appareil selon l'une quelconque des revendications 8 à 14 ; et
un processeur configuré pour corréler des signaux audio d'intensité acoustique, de signature spectrale et de durée spécifiques à des critères définissant un événement de miction et déterminer si un événement de miction s'est produit pour ledit animal d'élevage.

16. Système selon la revendication 15, ledit processeur étant une station centrale de traitement.
